# EUROPEAN PATENT APPLICATION

(11) **EP 4 821 474 A1**
(43) Date of publication of application: **14.10.2026**
(21) Application number: 24900805.3
(22) Date of filing: 27.09.2024
(51) Int. Cl.: A61H 1/02, A61N 1/36, A61N 1/04, A41D 13/00

(54) **HYBRID ACTUATOR**

(30) Priority: 04.12.2023 KR 20230173703
(71) Applicant: Korea Electronics Technology Institute, Bundang-gu, Seongnam-si Gyeonggi-do 13509 (KR)
(72) Inventor: WON, Yun Jae, Seongnam-si Gyeonggi-do 13436 (KR)
(74) Representative: Isarpatent
(86) International application number: PCT/KR2024/014728
(87) International publication number: WO 2025/121617

(57) **Abstract**

The present invention relates to a hybrid actuator which induces an operation of a target body part through electrical stimulation and enables a posture of a corresponding body part to be maintained using electrostatic force and tension. The hybrid actuator includes: a first frame fixed to a target body part; a second frame fixed to a body part different from the target body part; a stimulator which generates an electrical stimulation signal for stimulating muscles moving the target body part during a predetermined time; an electrostatic clutch which includes a first electrode and a second electrode and applies static electricity after the electrical stimulation signal is generated, to generate an electrostatic force between the first electrode and the second electrode; a first tension part which connects the first electrode and the first frame and enables a posture of the target body part to be maintained through tension; and a second tension part which connects the first electrode and the second frame and applies tension to the first electrode.

## Description

### [Technical Field]

The present invention relates to an actuator structure for causing movement of a specific body part and maintaining a posture after the movement is performed.

### [Background Art]

Patients with motor nerve disorders are continuously increasing due to congenital causes, genetic factors, unexpected accidents, falls and deterioration of physical functions of elderly people, spinal cord injuries, cerebrovascular diseases, strokes, and the like. The number of patients with motor nerve disorders in Korea increased from 1.8 million in 2004 to 3.4 million in 2020, and the number of patients with complete paralysis in Korea reached 458 thousand in 2020.

According to the results of research conducted at the University of California, Irvine, US (research by Kim D. Anderson), it was reported that the most desired body functions of patients with quadriplegia to restore are the movement functions of hands and arms.

Accordingly, there is an urgent need to develop an upper-limb assistive device or rehabilitation device that moves the hands or arms or assists movements in accordance with the intention of a patient with a motor nerve disorder or a patient with complete paralysis.

However, the upper-limb assistive device or rehabilitation device in the related art lacks portability due to factors such as requiring a stationary robotic arm, or is restricted to hospital use, and therefore there is a need to develop an upper-limb assistive device or rehabilitation device that is portable and usable both indoors and outdoors.

Further, for the upper-limb assistive device or rehabilitation device, there is a need for a function of recognizing a patient's movement intention to precisely control movement and maintaining a posture of an arm or a hand for a predetermined time after the control.

### [Detailed Description of Invention]

### [Technical Problem]

The present invention is directed to providing a hybrid actuator using electrostatic force and a loop that allows a posture of an arm or a hand of a wearer of an upper-limb assistive device or rehabilitation device to be maintained using an electrostatic effect appearing when a high voltage is applied between two electrodes, frictional force, and loop tension.

The object of the present invention is not limited to the object described above, and other objects that are not mentioned above will be clearly understood by those skilled in the art from the following description.

### [Technical Solution]

A hybrid actuator according to one embodiment of the present invention includes a first frame fixed to a target body part, a second frame fixed to a different body part from the target body part, a stimulator configured to generate an electrical stimulation signal for stimulating a muscle that moves the target body part for a predetermined time, an electrostatic clutch including a first electrode and a second electrode facing the first electrode and configured to apply static electricity for a predetermined time after generation of the electrical stimulation signal such that electrostatic force is generated between the first electrode and the second electrode, a first tension member configured to connect the first electrode to the first frame and maintain a posture of the target body part through tension between the first electrode and the first frame, and a second tension member configured to connect the first electrode to the second frame and apply tension to the first electrode.

In one embodiment of the present invention, the second electrode may be formed of a conductive fiber.

In one embodiment of the present invention, the second electrode may correspond to a portion of clothing worn by a user.

In one embodiment of the present invention, the second tension member may include a strain sensor.

In one embodiment of the present invention, the second tension member may measure a horizontal-direction position of the first electrode with respect to the second electrode.

In one embodiment of the present invention, in the electrostatic clutch, an amount of charge of the static electricity may vary according to a horizontal-direction position of the first electrode with respect to the second electrode.

In one embodiment of the present invention, the hybrid actuator may further include a restoring unit configured to restore, when application of the static electricity is released, the target body part to a posture prior to the generation of the electrical stimulation signal.

In one embodiment of the present invention, the first frame, the second frame, and the second electrode may be fixed to clothing worn by a user of the hybrid actuator.

A method of operating a hybrid actuator according to one embodiment of the present invention includes moving, by a hybrid actuator, a target body part by applying an electrical stimulation signal to a muscle of the target body part, maintaining, by the hybrid actuator, a posture of the target body part using electrostatic force and tension, and restoring, by the hybrid actuator, the posture of the target body part to a state before the electrical stimulation signal is applied by releasing the electrostatic force and reducing the tension.

In one embodiment of the present invention, the maintaining of the posture may include generating, by the hybrid actuator, the electrostatic force using an electrostatic clutch including a first electrode and a second electrode facing the first electrode.

In one embodiment of the present invention, the maintaining of the posture may include applying, by the hybrid actuator, the tension to the target body part using a first tension member connecting a first frame fixed to the target body part to the electrostatic clutch and a second tension member connecting the electrostatic clutch to a second frame fixed to a different body part from the target body part.

In one embodiment of the present invention, the second tension member may include a strain sensor.

### [Advantageous Effects]

According to one embodiment of the present invention, after controlling movement of an arm or a hand of a wearer of an assistive device through muscle stimulation, a posture and a shape of a corresponding part of the wearer are maintained using electrostatic force and a loop, thereby allowing a movement assistance function for the wearer to effectively operate.

The effects obtainable from the present invention are not limited to the effects mentioned above, and other effects that are not mentioned may be clearly understood by those of ordinary skill in the art to which the present invention belongs from the following description.

### [Description of Drawings]

FIG. 1 is a block diagram illustrating a configuration of a hybrid actuator according to one embodiment of the present invention.
FIG. 2 is a view illustrating a configuration of a hybrid actuator according to one embodiment of the present invention.
FIG. 3 is a reference view illustrating an example of controlling movement of a hand using a hybrid actuator according to the present invention.
FIG. 4 is a reference view illustrating an example of controlling movement of an arm using a hybrid actuator according to the present invention.
FIG. 5 is a block diagram illustrating a detailed configuration of a hybrid actuator according to one embodiment of the present invention.
FIG. 6 is a flowchart for describing a method of operating a hybrid actuator according to one embodiment of the present invention.

### [Modes of the Invention]

Advantages and features of the present invention, and methods of achieving the advantages and features will be clarified with reference to embodiments described below in detail together with the accompanying drawings. However, the present invention is not limited to the embodiments disclosed below but will be implemented in a variety of different forms. The present embodiments are only provided to allow disclosure of the present invention to be completed, and to completely inform those skilled in the art to which the present invention belongs of the scope of the invention, and the present invention is merely defined by scope of the claims. Meanwhile, the terms used in the present specification are for the purpose of describing the embodiments only and are not intended to limit the present invention. In the present specification, the singular forms are intended to include the plural forms as well, unless the context clearly indicates otherwise. As used in the present specification, "comprises" and/or "comprising" does not preclude the presence or addition of one or more other components, steps, operations, and/or elements in addition to the stated components, steps, operations, and/or elements.

While the terms "first," "second," etc., may be used herein to describe various elements, such elements should not be limited by the terms. The terms may be used only for the purpose of distinguishing one component from another component. For example, without departing from the scope of the present invention, a first component could be termed a second component and similarly, a second component could also be termed a first component.

It will be understood that when an element is referred to as being "coupled" or "connected" to another element, the element may be directly coupled or connected to the other element, or intervening elements may also be present. In contrast, it will be understood that when an element is referred to as being "directly coupled" or "directly connected" to another element, there are not intervening elements present. Other expressions describing relationships between components, such as "between" and "directly between" or "adjacent to" and "directly adjacent to," should be interpreted in the same manner.

When describing the present invention, when it is determined that a detailed description of related known technology may unnecessarily obscure the gist of the present invention, the detailed description thereof will be omitted.

Hereinafter, embodiments of the present invention will be described in detail with reference to the accompanying drawings. When describing the present invention, the same reference numerals will be used for the same elements regardless of drawing numbers in order to facilitate overall understanding of the present invention.

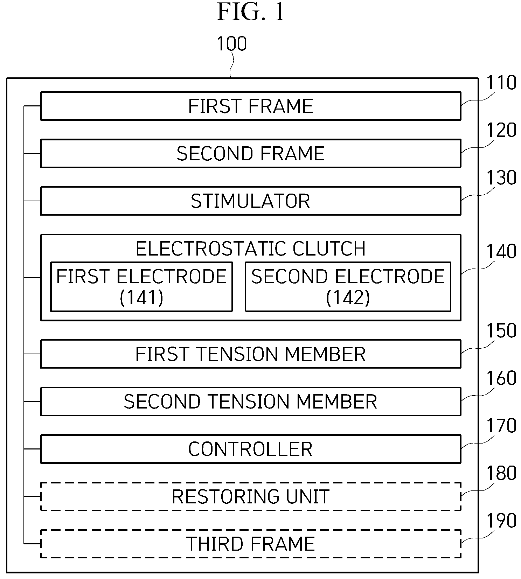

FIG. 1 is a block diagram illustrating a configuration of a hybrid actuator according to one embodiment of the present invention.

A hybrid actuator 100 according to one embodiment of the present invention induces movement of a target body part through electrical stimulation and maintains a posture of the target body part using electrostatic force and loop tension.

The hybrid actuator 100 according to one embodiment of the present invention includes a first frame 110, a second frame 120, a stimulator 130, an electrostatic clutch 140, a first tension member 150, a second tension member 160, and a controller 170, and may further include a restoring unit 180.

The first frame 110 is fixed to a body part (hereinafter referred to as a "target body part") that is a target required for posture support by operating the hybrid actuator 100. Specifically, the first frame 110 is fixed to skin or clothing of the target body part. Since the first frame 110 is fixed to the target body part, tension may be applied to the target body part through the first tension member 150 connected to the first frame 110, thereby allowing a posture of the target body part to be maintained.

The second frame 120 is fixed to a different body part from the target body part (skin or clothing). The second frame 120 serves to fix the hybrid actuator 100 to a specific part of the body of a wearer so that the hybrid actuator 100 does not shake. Accordingly, the hybrid actuator 100 may accurately operate the target body part and stably maintain a posture of the target body part.

The stimulator 130 includes a muscle stimulation electrode 131 and, according to a control command of the controller 170, generates an electrical stimulation signal for stimulating a muscle that moves the target body part through the muscle stimulation electrode 131 for a predetermined time, thereby causing the target body part to perform a predetermined movement. That is, the stimulator 130 stimulates muscle nerves by applying electrical current to the skin through the muscle stimulation electrode 131, thereby causing a muscle to contract such that the target body part performs a predetermined movement. A plurality of muscle stimulation electrodes 131 may be included in the stimulator 130 (when there are n electrodes, the electrodes are denoted as 131-1, 131-2, ..., 131-n). For example, the stimulator 130 causes a flexor muscle to contract by applying an electrical stimulation signal (current) to an arm portion through the muscle stimulation electrode 131, thereby allowing a specific finger to bend.

The electrostatic clutch 140 includes a first electrode 141 and a second electrode 142. The first electrode 141 faces the second electrode 142. The electrostatic clutch 140 applies static electricity to the first electrode 141 and the second electrode 142 under the control of the controller 180 after a point in time at which the stimulator 130 generates the electrical stimulation signal, such that electrostatic force is generated between the two electrodes. The second electrode 142 may be formed of conductive fiber and may be fixed to clothing worn by a user or may be a portion of the clothing worn by the user.

The first tension member 150 connects the first frame 110 to the first electrode 141 and applies tension to the first frame 110 to maintain a posture of the target body part. For example, the first tension member 150 may be any one of various elastic materials having tension such as a loop, a wire, a spring, a rubber band, a flexible ribbon, and the like.

The second tension member 160 connects the second frame 120 to the first electrode 141 and applies tension to the first electrode 141 such that an area of the first electrode 141 facing the second electrode 142 is greater than or equal to a reference value. Accordingly, the second tension member 160 serves to support the maintenance of the electrostatic force between the first electrode 141 and the second electrode 142 when the posture of the target body part needs to be maintained. The second tension member 160 may also be any one of elastic materials having tension, like the first tension member 150.

In particular, the second tension member 160 may include a strain sensor. In this case, the strain sensor serves to apply tension to the first electrode 141 and to measure a position. That is, when the second tension member 160 includes the strain sensor, in addition to the role of applying tension to the first electrode 141, the second tension member 160 measures a distance between the second frame 120 and the first electrode 141, in other words, a horizontal-direction position of the first electrode 141 with respect to the second electrode 142 and transmits the measured distance to the controller 170. The controller 170 controls the amount of charge of the static electricity applied to the electrostatic clutch 140 according to the horizontal-direction position of the first electrode 141 with respect to the second electrode 142 such that tension required for maintaining the posture of the target body part is maintained. That is, the electrostatic clutch 140 has a characteristic in which the amount of static electricity varies according to the horizontal-direction position of the first electrode 141.

As another example, the first tension member 150 or the second tension member 160 may be made of a shape memory alloy (e.g., a nickel-titanium alloy). In this case, the controller 170 may increase the temperature of the shape memory alloy by applying an electric current thereto, thereby causing the shape memory alloy to contract. When the shape memory alloy is used for the first tension member 150 or the second tension member 160, the stimulator 130 may be omitted in the hybrid actuator 100.

The controller 170 controls the stimulator 130 and the electrostatic clutch 140 according to a signal received from an external device. The controller 170 transmits a control command to the stimulator 130 such that the stimulator 130 generates the electrical stimulation signal for a predetermined time and controls the electrostatic clutch 140 after generation of the electrical stimulation signal such that the electrostatic force between the first electrode 141 and the second electrode 142 is generated for a predetermined time, thereby maintaining the posture of the target body part. For example, the controller 170 includes a brain neural signal interface and receives a brain neural signal or a signal converted therefrom and transmits a control command to the stimulator 130 according to the received signal such that the stimulator 130 generates the electrical stimulation signal for a predetermined time, thereby allowing a finger, which is the target body part, to bend.

The restoring unit 180 restores, when application of the static electricity of the electrostatic clutch 140 is released, the target body part to a posture prior to the generation of the electrical stimulation signal. The restoring unit 180 connects the first frame 110 to a third frame 190 and applies tension to the first frame 110 such that the target body part is restored to the posture prior to the generation of the electrical stimulation signal. The restoring unit 180 may be any one of various elastic materials having tension such as a loop, a wire, a spring, a rubber band, a flexible ribbon, and the like. The third frame 190 is fixed to a specific part of the body or clothing. The third frame 190 is disposed to apply tension to the first frame 110 in a direction opposite to a direction of tension between the first frame 110 and the first electrode 141.

Meanwhile, specific shapes of the first frame 110, the second frame 120, and the third frame 190 may vary. For example, when the target body part is a finger, the first frame 110 may be a thimble worn on a fingertip by the user, the second frame 120 may be a fixing device included in an exoskeleton or a wearable suit worn by the user, and the third frame 130 may be a fixing device mounted on a glove worn by the user.

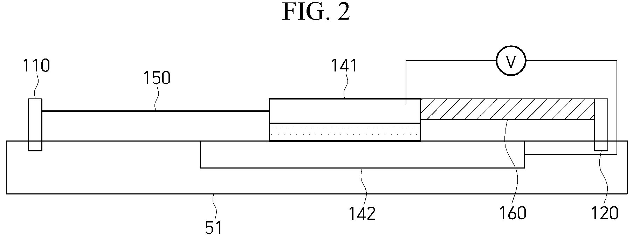

FIG. 2 is a view illustrating a configuration of a hybrid actuator according to one embodiment of the present invention. As illustrated in FIG. 2, a first frame 110 and a first electrode 141 are connected by a first tension member 150, and the first electrode 141 is connected to a second frame 120 by a second tension member 160. The second tension member 160 may be a strain sensor or may include the strain sensor. The first electrode 141 faces a second electrode 142 and is preferably parallel thereto. In FIG. 2, the second electrode 142 is formed of a conductive fiber material and is fixed to clothing 51. When a posture of a target body part needs to be maintained, it is preferable that an area of the first electrode 141 facing the second electrode 142 be as large as possible. The controller 170 controls the amount of charge of the static electricity between the first electrode 141 and the second electrode 142 based on a measured position of the first electrode 141 such that tension of the first tension member 150 is maintained at a constant level.

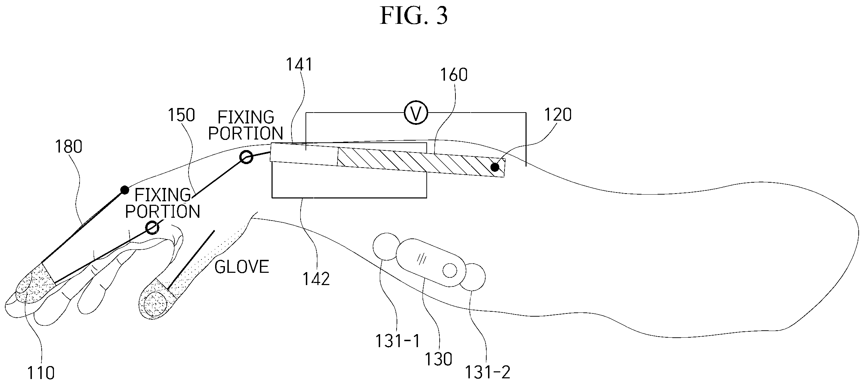

FIG. 3 is a reference view illustrating an example of controlling movement of a hand using a hybrid actuator according to the present invention. In FIG. 3, a target body part to be controlled by the hybrid actuator 100 is a finger, and the first frame 110 is fixed to a fingertip.

The first tension member 150 connects the first frame 110 to the first electrode 141, and a specific point of the first tension member 150 is fixed to a fixture worn by a user. For example, the first tension member 150 connects the first frame 110 to the first electrode 141 in a manner such that the first tension member 150 passes through a hole of a glove worn by a user. The hole serves to prevent a central portion of the first tension member 150 from deviating from a user's body to a predetermined range or more.

In order to bend a user's finger according to an intention of the user (e.g., a brain neural signal), the controller 170 applies an electrical stimulation signal to a flexor muscle through the stimulator 130 attached to a forearm of the user. Further, the controller 170 controls the electrostatic clutch 140 so that electrostatic force is generated between the first electrode 141 and the second electrode 142. The posture of the target body part is maintained using the tension of the first tension member 150 and the second tension member 160 and the electrostatic force of the electrostatic clutch 140, as the stimulator 130 may not continuously generate the electrical stimulation signal due to factors such as a user's safety and fatigue and battery capacity.

The restoring unit 180 connects the first frame 110 to the third frame 190, and when the tension between the first electrode 141 and the first frame 110 is reduced due to release of the electrostatic force, applies tension in an opposite direction so that the finger, which is the target body part, is restored to a position prior to the generation of the electrical stimulation signal.

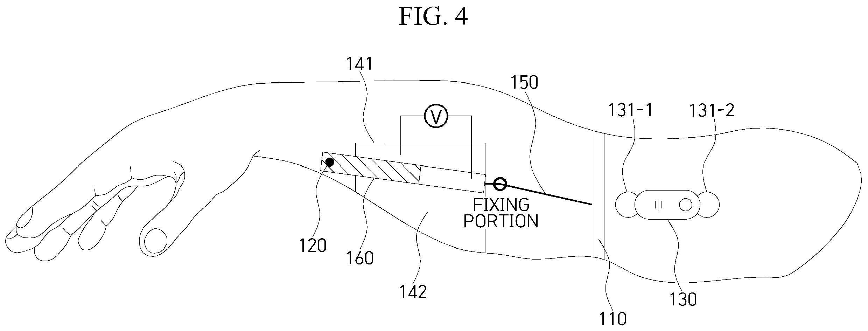

FIG. 4 is a reference view illustrating an example of controlling movement of an arm using the hybrid actuator according to the present invention. Unlike the first frame 110 is disposed at the fingertip in FIG. 3, the first frame 110 illustrated in FIG. 4 is disposed at a forearm to control movement of an arm. The second frame 120 is disposed at a position close to a hand. In this way, positions of components of the hybrid actuator 100 may vary according to a target body part to be controlled.

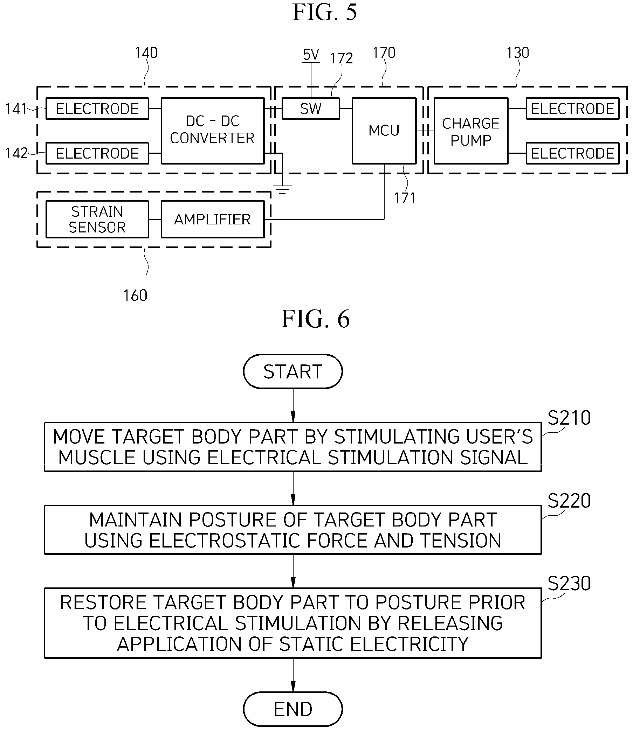

FIG. 5 is a block diagram illustrating a detailed configuration of a hybrid actuator according to one embodiment of the present invention.

In the embodiment of FIG. 5, a controller 170 includes a microcontroller unit (MCU) 171 and a switch 172. The MCU 171 may receive a signal from outside through a separate communication module. The MCU 171 may generate a control command based on an external signal. The MCU 171 transmits the control command to the stimulator 130 such that the stimulator 130 generates an electrical stimulation signal and applies static electricity to electrodes 141 and 142 of the electrostatic clutch 140 through the switch 172 such that electrostatic force is generated between the electrodes 141 and 142. The MCU 171 determines a relative position of the first electrode 141 with respect to the second electrode 142 based on a sensing signal transmitted by the strain sensor 160 and controls the amount of charge of the static electricity applied to the electrostatic clutch 140 according to the determined position.

FIG. 6 is a flowchart for describing a method of operating a hybrid actuator according to one embodiment of the present invention. It is assumed that the method of operating a hybrid actuator is performed by the hybrid actuator 100.

As illustrated in FIG. 6, the method of operating a hybrid actuator according to one embodiment of the present invention includes operations S210 to S230.

Operation S210 is an operation of moving a target body part. The controller 170 generates a control command according to a signal received from an external device and transmits the control command to the stimulator 130. The stimulator 130 includes a muscle stimulation electrode 131 and, according to a control command of the controller 170, generates an electrical stimulation signal for stimulating a muscle that moves the target body part through the muscle stimulation electrode 131 for a predetermined time, thereby causing the target body part to perform a predetermined movement.

Operation S220 is an operation of maintaining a posture of the target body part. After generation of the electrical stimulation signal, the electrostatic clutch 140 applies static electricity to the first electrode 141 and the second electrode 142 under control of the controller 170 such that electrostatic force is generated between the electrodes. In addition,

the second tension member 160 is fixed to the second frame 120 and applies tension to the first electrode 141, and the first tension member 150 connected to the first electrode 141 and the first frame 110 applies tension to the first frame 110 fixed to the target body part. The hybrid actuator 100 maintains the posture of the target body part using the electrostatic force generated in the electrostatic clutch 140 and the tension of the first tension member 150 and the second tension member 160.

Operation S230 is an operation of restoring the posture of the target body part. When a predetermined time elapses, the static electricity of the electrostatic clutch 140 is released under control of the controller 170. As the static electricity is released, a constrained state of the first electrode 141 is released, and the tension of the first tension member 150 and the second tension member 160 is reduced. The restoring unit 180 restores the target body part to a posture prior to the generation of the electrical stimulation signal when application of the static electricity of the electrostatic clutch 140 is released. The restoring unit 180 having both ends fixed to the first frame 110 and the third frame 190 applies tension to the first frame 110 such that the target body part is restored to the posture prior to the generation of the electrical stimulation signal.

Even when omitted in the description with reference to FIG. 6, descriptions made with reference to FIGS. 1 to 5 may be applied to FIG. 6. Descriptions made with reference to FIG. 6 may also be applied to FIGS. 1 to 5.

Although exemplary embodiments of the present invention have been described above, it is understood that those skilled in the art may make various changes and modifications to the present invention without departing from the spirit and scope of the present invention set forth in the claims below.

## Claims

1. A hybrid actuator comprising:
a first frame fixed to a target body part;
a second frame fixed to a different body part from the target body part;
a stimulator configured to generate an electrical stimulation signal for stimulating a muscle that moves the target body part for a predetermined time;
an electrostatic clutch including a first electrode and a second electrode facing the first electrode and configured to apply static electricity for a predetermined time after generation of the electrical stimulation signal such that electrostatic force is generated between the first electrode and the second electrode;
a first tension member configured to connect the first electrode to the first frame and maintain a posture of the target body part through tension between the first electrode and the first frame; and
a second tension member configured to connect the first electrode to the second frame and apply tension to the first electrode.

2. The hybrid actuator of claim 1, wherein the second electrode is formed of a conductive fiber.

3. The hybrid actuator of claim 1, wherein the second electrode corresponds to a portion of clothing worn by a user.

4. The hybrid actuator of claim 1, wherein the second tension member includes a strain sensor.

5. The hybrid actuator of claim 1, wherein the second tension member measures a horizontal-direction position of the first electrode with respect to the second electrode.

6. The hybrid actuator of claim 1, wherein, in the electrostatic clutch, an amount of charge of the static electricity varies according to a horizontal-direction position of the first electrode with respect to the second electrode.

7. The hybrid actuator of claim 1, further comprising a restoring unit configured to restore, when application of the static electricity is released, the target body part to a posture prior to the generation of the electrical stimulation signal.

8. The hybrid actuator of claim 1, wherein the first frame, the second frame, and the second electrode are fixed to clothing worn by a user of the hybrid actuator.

9. A method of operating a hybrid actuator, comprising:
moving, by a hybrid actuator, a target body part by applying an electrical stimulation signal to a muscle of the target body part;
maintaining, by the hybrid actuator, a posture of the target body part using electrostatic force and tension; and
restoring, by the hybrid actuator, the posture of the target body part to a state before the electrical stimulation signal is applied by releasing the electrostatic force and reducing the tension.

10. The method of claim 9, wherein the maintaining of the posture includes generating, by the hybrid actuator, the electrostatic force using an electrostatic clutch including a first electrode and a second electrode facing the first electrode.

11. The method of claim 10, wherein the maintaining of the posture includes applying, by the hybrid actuator, the tension to the target body part using a first tension member connecting a first frame fixed to the target body part to the electrostatic clutch and a second tension member connecting the electrostatic clutch to a second frame fixed to a different body part from the target body part.

12. The method of claim 11, wherein the second tension member includes a strain sensor.
